# EUROPEAN PATENT SPECIFICATION

(11) **EP 2 720 005 B1**
(45) Date of publication and mention of the grant of the patent: **25.05.2022**
(21) Application number: 12187950.6
(22) Date of filing: 10.10.2012
(51) Int. Cl.: G01F 1/50, A61B 5/087, A61B 5/08, A61M 16/08, A61M 16/10, A61M 16/00

(54) **Arrangement for a pressure measurement of a breathing gas flowing along a flow channel**
Anordnung für eine Druckmessung eines entlang eines Durchflusskanals fließenden Atemgases
Agencement pour une mesure de pression d'un gaz de respiration s'écoulant le long d'un canal d'écoulement

(43) Date of publication of application: 16.04.2014
(73) Proprietor: General Electric Company, Schenectady, NY 12345 (US)
(72) Inventor: Hietala, Mika, 02610 Espoo (FI)
(74) Representative: Fennell, Gareth Charles

(56) References cited:
- EP-A1- 2 017 586
- DE-B- 1 062 021
- FR-A5- 2 079 983
- US-A- 3 267 729
- US-A- 4 599 906
- US-B1- 6 959 610
- DATABASE WPI Week 201017 Thomson Scientific, London, GB; AN 2010-C24488 XP002693574, -& CN 201 402 160 Y (NANJING HWAPON TECHNOLOGY CO LTD) 10 February 2010 (2010-02-10)
- None

## Description

### BACKGROUND OF THE INVENTION

This disclosure relates generally to an arrangement for a pressure measurement of a breathing gas flowing along a flow channel.

US-A-4 599 906 discloses a pressure transducer assembly for measuring differential pressures across a restriction in a pipe carrying process fluids such as waste water, pulp stock and light slurries. US 3,267,729 is concerned with a compensating device for gas flowmeters.

EP 2 017 586 relates to a monitor for surveying measured values that are indicative of a person's breathing.

In hospitals, during intensive care and operations, a respiratory apparatus must mostly be used to take care of a patient's respiration. An unhindered flow of gases into and out of the patient's lungs is naturally of vital importance. A condition of gas channels can be monitored both by measuring concentrations of inhaled and exhaled gases and by measuring a flow and pressure of the gases. Especially, monitoring of the carbon dioxide content of an exhalation gas is widely used as a routine in operating theaters and now more frequently also in the intensive care unit. However, the flow and pressure measurements are an important additional function both in respect of safety and because they make it possible to calculate quantities descriptive of the mechanical operation and respiratory metabolism of the lungs.

In principle, there are many applicable types of flow sensors. However, measurements under clinical conditions involve many problems. The flow is measured from the end of a so-called intubation tube inserted into the patient's windpipe. The sensor is therefore exposed to humidity, condensed water, and mucous secretions coming from the windpipe. It is clear that such soiling is likely to affect the operation of most types of flow sensors. The main types of flow detector and their principles are presented e.g. in the publication Doebelin: Measurement Systems, McGraw-Hill Kogakusha, 1976. Flow sensors based on differential pressure are best suited for clinical use. The flow in the respiratory tube may be laminar or turbulent. In the case of laminar flow, the pressure difference across a flow restricting element placed in the tube is directly proportional to the flow. In the case of a turbulent flow, the pressure difference depends on the square of the flow. In practice, the relation between the measured pressure difference and the flow always has to be empirically determined for a specific mechanical sensor construction. The flow sensors currently used are generally made of plastic and can either be disposable or reusable. Because a sensitive differential pressure sensor is fragile it is normally positioned away from the flow sensor tube and is connected to it using plastic tubing. In order to zero the differential pressure sensor and to increase its accuracy zeroing valves are included. They have to be small and, therefore, add restrictors to the pneumatic circuit. The airway pressure is typically measured using a separate pressure gauge connected to one side of the differential pressure sensor. Because of the internal volume of the pressure gauge and also volume differences prior to the zeroing valves the complete pneumatic circuit becomes non-symmetrical. This has no effect on slow pressure and flow measurements but fast pressure fluctuation and high respiration rates would be measured with distortion. Especially for neonatal respiratory measurements a fast response time is crucial.

One remedy is to keep all volumes as small as possible and to avoid using restrictors. Unfortunately, this is not possible in a patient spirometry circuit. It could be possible to position the pressure sensors close to the respiratory tube but there is then always the risk for contamination by water and mucus. Additionally, the sensor temperature would fluctuate because of the warm respiratory air flow. A safer place for the pressure sensors is in or close to the patient gas analyzer. However, this means that long tubing has to be used to transfer the pressure from the airway to the pressure sensor. This would add restrictors and volumes to the pneumatic circuit and, as a result, the high frequency response could be degraded.

### BRIEF DESCRIPTION OF THE INVENTION

The present invention provides an arrangement as defined in claim 1.

The above-mentioned shortcomings, disadvantages and problems are addressed herein which will be understood by reading and understanding the following specification.

Various other features, objects, and advantages of the invention will be made apparent to those skilled in art from the accompanying drawings and detailed description thereof.

### BRIEF DESCRIPTION OF THE DRAWINGS

Figure 1 is a general schematic diagram of an arrangement for a pressure measurement of a breathing gas in accordance with an embodiment;
Figure 2 is a perspective view of a body of an arrangement for a pressure measurement of a breathing gas in accordance with an embodiment; and
Figure 3 is a perspective view of an arrangement for a pressure measurement of a breathing gas shown in Figure 1 in an operating environment.

### DETAILED DESCRIPTION OF THE INVENTION

Specific embodiments are explained in the following detailed description making a reference to accompanying drawings. These detailed embodiments can naturally be modified and should not limit the scope of the invention as set forth in the claims.

In accordance with an embodiment an arrangement with a pressure measurement made of a breathing gas flowing along a flow channel with relatively fast response time for both pressure and flow is presented.

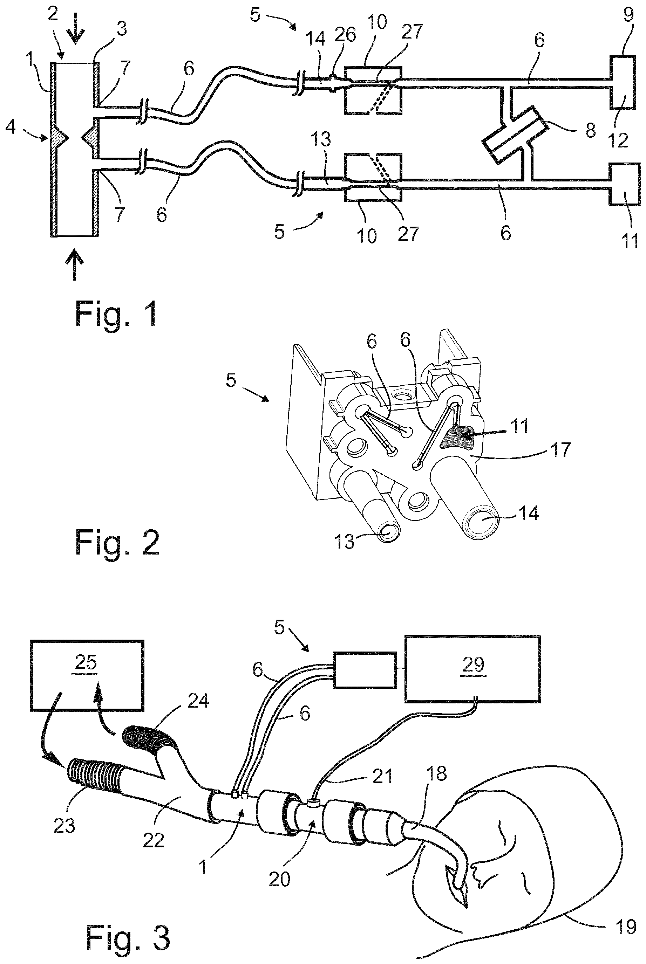

Figure 1 presents a general schematic diagram for a pressure measurement, especially a breathing gas pressure measurement, either can be measured a differential pressure or an airway pressure. The differential pressure of a gas flow can be measured over a flow restriction indicating a flow rate. The arrangement for measuring the differential pressure may comprise a separate flow adapter or the flow adapter is an integral part of a channel conveying a breathing gas. The flow adapter can also be used to provide a signal indicative of the airway pressure, in which case the airway gas pressure is compared to outside pressure.

An arrangement presented in Figure 1 is for a pressure measurement of a breathing gas, especially for improving an accuracy of a pressure measurement made of a breathing gas. The arrangement may comprise a flow adapter 1 with a tubular flow channel 2 for passing a gas flow, such as breathing gas to be measured. The flow adapter can also be a separate product and not necessarily part of the arrangement. The flow can be in either direction depending on the breathing phase, either an inspiratory or an expiratory breathing phase. The flow channel 2 is delimited by a wall 3 and it is provided with a restricting element 4 to restrict the gas flow in flow channel 2. Communicating with the flow channel 2 on either side of the restricting element 4 are at least two separate branch lines 5, which may be directly or indirectly connectable to the flow channel 2. Also the flow channel or the flow adapter may be integrated with the branch lines or only with a part of the branch lines. Typically at least two branch lines 5 may be in pneumatic communication with the flow channel or the flow adapter.

Each branch line 5 has a predetermined gas volume and comprises a measuring channel 6 connected to both sides of the restricting element 4 transmitting pressure signals from the flow channel through a measuring channel input 7 to the measuring channel for providing a signal indicative of the pressure in the flow channel and based on which signal the pressure difference and/or the airway pressure can be determined. The pressure difference created in the flow channel by the action of the flow restricting element 4 can be used to calculate the flow rate. The measuring channels 6 are normally plastic tubing with suitable connectors in one or both ends. The length of these channels may be up to 3 meters or even 6 meters but could also be shorter. In the former case at least part of branch lines 5 could be situated inside a gas analyzer 29 shown in Figure 3, in the latter case it could be closer to the flow adapter 1 in vicinity to a so called mainstream sensor.

The at least two branch lines comprise at least one sensor 8 and/or 9. Depending on the sensor type it is in pneumatic communication with the measuring channel of at least one of the two branch lines 5 and along them with the flow channel to provide a signal indicative of the pressure in the flow channel 2. In Figure 1 the sensor 8 is a differential pressure sensor and the sensor 9 is a pressure gauge. The differential pressure sensor is in pneumatic communication with measuring channels of both two separate branch lines 5 to obtain a signal indicative of the breathing gas flow rate of the flow channel. The pressure gauge is in pneumatic communication with the measuring channel in at least one of the separate branch lines 5 to obtain a signal indicative of an airway pressure of the flow channel.

Each of the branch lines 5 further may comprise a main restrictor 27 of internal pneumatic components. The main restrictor is normally part of the zeroing valve, but it is typically present without the zeroing valve because all components may cause restriction. Each of the at least two branch lines should advantageously have substantially identical restriction.

Additionally there may be electronics like amplifiers and a CPU unit to operate the sensors and valves and to calculate the flow from the differential pressure given by sensor 8 and the airway pressure from the pressure gauge 9. The electronics may also be part of an analyzer 29.

The pneumatic arrangement of the at least two branch lines 5 comprising measuring channels 6, the commercial sensors and valves has an inherent pneumatic response time to sudden pressure and flow changes in flow channel 2. In order to increase accuracy of the measurement this response time must be pneumatically optimized. The measuring channels 6, also named spirometry tubing, can be made to have large enough inner diameter to transfer the pressures well. With the branch lines 5 there are normally two zeroing valves 10, one zeroing valve for each of at least two branch lines. These are used to zero the pressure sensors to ambient pressure. This is very important especially for the sensor 8, which is in pneumatic communication with the measuring channel input 7 through the zeroing valve, indicating pressure difference over the restricting element 4 because the lower the measurable pressure difference, the lower the measurable flow will be. Typically, the operational pressure of a differential pressure sensor can be ± 12.5 mbar. Operating both zeroing valves 10 momentarily will disconnect the pressure sensors from the measuring circuit and connect them to ambient air. This may be repeated e.g. every minute or 2 minutes to maintain the measuring accuracy.

Especially the zeroing valve 10 is normally also a main restrictor 27 in the system because of its required small size. With main restrictor is understood the major part, at least 90 % of the restriction within the pneumatic system. The main restrictor could also comprise other parts of the pneumatic circuit, e.g. a possible water filter in the measuring channel 6. The total dimensions of the branch lines 5 should be kept as small as possible, still using commercially obtainable components for economical reasons. Its size without measuring channels 6 may be close to a cube with 25 mm sides. After the zeroing valve 10 there is the sensor 9, such as a pressure gauge or equivalent in one branch line.

The arrangement also comprises a balancing volume 11 in pneumatic communication with the other branch line without the pressure gauge communication, because the pressure gauge has a certain internal volume 12, which can be compensated by this balancing volume. Thus the balancing volume may be directly or indirectly connectable to such a branch line having a predetermined volume less than the predetermined volume of another of the at least two branch lines. Also it is possible to integrate the balancing volume to such branch line. Without the balancing volume 11, both branch lines 5 would have different volumes and would behave slightly differently for fast pressure changes. The main restrictor 27 and the volume 11 constitute a pneumatic low pass filter and the pressure curve shape will be different at high ventilation rates in each branch lines 5. Because the flow is calculated from a measured differential pressure, the output curve shape would be partly corrupted and the measurement result has an extra error. The problem can be avoided by adjusting the volumes of both branch lines 5 to substantially the same or advantageously equal. This can be achieved by adding the small balancing volume 11, such as a gas chamber, to the measurement branch without the sensor 9. The balancing volume in pneumatic communication with at least one of the branch lines is needed to substantially equalize the predetermined gas volumes of the at least two branch lines. To substantially equalize the predetermined gas volumes of the at least two branch lines the gas volume of the branch line having a predetermined volume less than the predetermined volume of another of the at least two branch lines is added resulting in the gas volume varying less than 10 %, more specifically less than 5 % or even more specifically less than 1 % from the another branch line. The volume size is the volume difference between the two branch lines 5 after the zeroing valve 10, which is succeeding the zeroing valves 10, assuming that the two zeroing valves are mechanically identical, having substantially the same restriction, this being the main restriction 27.

The balancing volume 11 will be placed near the sensor 8 providing the signal indicative of the pressure difference. A typical size of the balancing volume 11 is about 0.1 cubic centimeter, depending on the internal volume 12 of the sensor 9 in use. As shown in Figure 1 the sensor 8 providing the signal indicative of the pressure difference does not have to be exactly in a symmetrical position but the measuring channels 6 succeeding the zeroing valves 10, which are between the zeroing valves 10 and either the sensors 8, 9 in one of the branch lines or the sensor 8 and the balancing volume 11 in the another branch line, are kept close to the same. There may also be other restrictors in the pneumatic circuit but, in most cases, they are similar in both measuring channels 5 succeeding the main restrictors 27 or the zeroing valves 10 representing minor restrictions, which may be about 10 % of the restrictions in the arrangement. Therefore, to balance the pneumatic time constant it is easier to balance the volumes on either side of the main restriction 27.

Also input volumes 13 and 14 preceding the zeroing valves 10 between the measuring channel inputs 7 and one of the zeroing valves 10 having the main restrictor 27, including possible connectors, should be balanced because they influence the pneumatic time constant for reverse flow in the flow channel 2 or subsequent negative pressure difference as measured by the sensor 8 providing the signal indicative of the pressure difference. The volumes are also kept as small as possible because high volume after the main restriction 27 makes the low-pass filter corner frequency to be lower than with a smaller volume. In case of differences in the volumes in both measuring channels 6 preceding the zeroing valves 10 or the main restrictors 27, between the zeroing valve and the measuring channel input 7, a balancing volume 26 may be added to such a branch line having a smaller volume so that the volumes on either sides of the measuring channels, but preceding the zeroing valves 10 or the main restrictors 27, are substantially identical. This volume balancing on either side of the main restrictors 27 is especially important when trying to measure high ventilation rates, up to 70 breaths/min or even higher.

Figure 2 shows in perspective a body part of the arrangement having the two branch lines 5 without the sensors 8, 9 and the zeroing valves 10. The input volumes 13 and 14 are in the form of conventional male and female connectors, respectively. The small balancing volume 11 can be seen as a cavity and also visible are the measuring channels 6 succeeding the zeroing valves with their connections to the pressure sensors 8, 9 and the zeroing valves 10. The surface 17 will be covered and sealed in the completed device.

Figure 3 presents a perspective view of an arrangement for a pressure and flow measurement shown in Figure 1 in an operating environment where an intubation tube 18 is inserted into the windpipe of a patient 19. Connected to a respiratory circuit is a flow adapter 1 as shown in detail in Figure 1. A connecting piece 20, provided with a sampling tube 21 for the analysis of gas, e.g. measurement of its concentration, is connected between the intubation tube 18 and a Y-shaped piece 22 connecting the inlet and outlet hoses 23, 24 of an apparatus 25 maintaining a respiration. The connecting piece 20 is normally so connected that it lies closest to the patient 19, but it could also be integrated with the flow adapter 1 or it could be placed between the flow adapter 1 and the Y-shaped piece 22. The gas sampling tube 21 is connected to the gas analyzer 29, in which the gas is measured and the signal is processed so as to produce a display (not shown) showing the variations in the gas concentration under measurement as a function of time, i.e. the respiration curve or concentration readings during inhalation and exhalation. The flow adapter 1 is also connected via a branch lines 5 to the gas analyzer 29, in which the signal is processed as to produce a display of the flow and pressure readings for inhalation and exhalation and possible other quantities derived from them. At least part of branch lines 5 may also be placed in the gas analyzer 29 and the gas concentration measurement can be performed in the connecting piece 20 or as integrated in with the flow adapter 1, if the device is a so-called mainstream gas sensor.

The written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

## Claims

1. An arrangement for a pressure measurement of a breathing gas flowing along a flow channel (2), the arrangement comprising:
first and second branch lines (5) configured to be in pneumatic communication with said flow channel, each of said first and second branch lines having a predetermined gas volume and each of said first and second branch lines comprising a measuring channel (5) for transmitting gaseous pressure signals from said flow channel,
wherein said first branch line comprises at least one sensor (9) in pneumatic communication with said measuring channel of the first branch line for providing a signal indicative of a pressure in said flow channel, wherein the at least one sensor (9) comprises a pressure gauge operable to obtain a signal indicative of an airway pressure in said flow channel (2), said arrangement further comprising a differential pressure sensor being in pneumatic communication with said measuring channels of said first and second branch lines to obtain a signal indicative of a breathing gas flow rate in said flow channel (2),
**characterized in that**
said second branch line comprises a balancing volume (11) in pneumatic communication with the second branch line for equalizing said predetermined gas volumes of said first and second branch lines.

2. The arrangement of claim 1, wherein said balancing volume is configured to be one of integrated with and either directly connectable to or indirectly connectable to said second branch line and said second branch line has a predetermined volume less than the predetermined volume of said first branch line.

3. The arrangement of claim 2, wherein said balancing volume equalizing said predetermined gas volumes of said first and second branch lines is configured to add the gas volume to said second branch line resulting in the gas volume varying less than 10 %, more specifically less than 5 % or even more specifically less than 1 % from said first branch line.

4. The arrangement of claim 1, wherein said balancing volume is a gas chamber in pneumatic communication with said second branch line.

5. The arrangement of claim 1, further comprising the flow channel (2), wherein the flow channel (2) comprises a flow adapter (1) having a restricting element (4) to restrict the flow through said adapter, said measuring channels being connected to both sides of said restricting element via respective measuring channel inputs (7).

6. The arrangement of claim 1 or 5, wherein the differential pressure sensor is for obtaining a signal indicative of a breathing gas flow in said flow channel (2) and the pressure gauge is for obtaining a signal indicative of an airway pressure in said flow channel (2).

7. The arrangement of claim 6, wherein said balancing volume is for compensating for an internal volume (12) of said pressure gauge.

8. The arrangement of claim 1, wherein said first and second branch lines (5) also comprise at least one internal pneumatic component having a main restriction (27), and wherein said main restriction (27) is a zeroing valve (10) for zeroing said sensor to ambient pressure.

9. The arrangement of claim 8, wherein said second branch line has a smaller volume in said measuring channel preceding said zeroing valve than the first branch line.

10. The arrangement of claim 8, wherein said sensor is in pneumatic communication with said flow channel (2) through said zeroing valve.

11. The arrangement of claim 8, wherein each of said at least two branch lines (5) have an identical main restriction (27).

## Patentansprüche

1. Anordnung zur Druckmessung eines Atemgases, das durch einen Strömungskanal (2) strömt, wobei die Anordnung umfasst:
eine erste und eine zweite Zweigleitung (5), die dafür ausgelegt sind, mit dem Strömungskanal in pneumatischer Verbindung zu stehen, wobei die erste und die zweite Zweigleitung jeweils ein vorbestimmtes Gasvolumen aufweisen und die erste und die zweite Zweigleitung jeweils einen Messkanal (5) zum Senden von Gasdrucksignalen aus dem Strömungskanal umfassen,
wobei die erste Zweigleitung mindestens einen Sensor (9) in pneumatischer Verbindung mit dem Messkanal der ersten Zweigleitung zum Bereitstellen eines Signals umfasst, das einen Druck im Strömungskanal angibt, wobei der mindestens eine Sensor (9) einen Druckmesser umfasst, der zum Erhalten eines Signals betreibbar ist, das einen Atemwegsdruck im Strömungskanal (2) angibt, wobei die Anordnung ferner einen Differenzdrucksensor, der in pneumatischer Verbindung mit den Messkanälen der ersten und der zweiten Zweigleitung steht, zum Erhalten eines Signals, das eine Atemgasströmungsrate im Strömungskanal (2) angibt, umfasst,
**dadurch gekennzeichnet, dass**
die zweite Zweigleitung ein Ausgleichsvolumen (11), das in pneumatischer Verbindung mit der zweiten Zweigleitung steht, zum Ausgleichen der vorbestimmten Gasvolumina der ersten und der zweiten Zweigleitung umfasst.

2. Anordnung nach Anspruch 1, wobei das Ausgleichsvolumen dafür ausgelegt ist, eines von in die zweite Zweigleitung integriert und entweder direkt mit ihr verbunden oder indirekt mit ihr verbunden zu sein, und die zweite Zweigleitung ein vorbestimmtes Volumen aufweist, das kleiner ist als das vorbestimmte Volumen der ersten Zweigleitung.

3. Anordnung nach Anspruch 2, wobei das Ausgleichsvolumen, das die vorbestimmten Gasvolumina der ersten und der zweiten Zweigleitung ausgleicht, dafür ausgelegt ist, das Gasvolumen der zweiten Zweigleitung hinzuzufügen, was dazu führt, dass das Gasvolumen weniger als 10 %, spezifischer weniger als 5 % oder noch spezifischer weniger als 1 % von der ersten Zweigleitung abweicht.

4. Anordnung nach Anspruch 1, wobei das Ausgleichsvolumen eine Gaskammer ist, die in pneumatischer Verbindung mit der zweiten Zweigleitung steht.

5. Anordnung nach Anspruch 1, ferner umfassend den Strömungskanal (2), wobei der Strömungskanal (2) einen Strömungsadapter (1) mit einem Drosselelement (4) zum Drosseln der Strömung durch den Adapter umfasst, wobei die Messkanäle mit beiden Seiten des Drosselelements über entsprechende Messkanaleingänge (7) verbunden sind.

6. Anordnung nach Anspruch 1 oder 5, wobei der Differenzdrucksensor dazu dient, ein Signal zu erhalten, das eine Atemgasströmung im Strömungskanal (2) angibt, und der Druckmesser dazu dient, ein Signal zu erhalten, das einen Atemwegsdruck im Strömungskanal (2) angibt.

7. Anordnung nach Anspruch 6, wobei das Ausgleichsvolumen zur Kompensation eines Innenvolumens (12) des Druckmessers dient.

8. Anordnung nach Anspruch 1, wobei die erste und die zweite Zweigleitung (5) außerdem mindestens eine interne pneumatische Komponente mit einer Hauptdrossel (27) umfassen und wobei die Hauptdrossel (27) ein Nullstellungsventil (10) zum Nullstellen des Sensors auf Umgebungsdruck ist.

9. Anordnung nach Anspruch 8, wobei die zweite Zweigleitung im Messkanal vor dem Nullstellungsventil ein kleineres Volumen aufweist als die erste Zweigleitung.

10. Anordnung nach Anspruch 8, wobei der Sensor über das Nullstellungsventil in pneumatischer Verbindung mit dem Strömungskanal (2) steht.

11. Anordnung nach Anspruch 8, wobei jede der mindestens zwei Zweigleitungen (5) eine identische Hauptdrossel (27) aufweist.

## Revendications

1. Agencement pour une mesure de pression d'un gaz respiratoire s'écoulant le long d'un canal d'écoulement (2), l'agencement comprenant :
des première et seconde conduites de ramification (5) configurées pour être en communication pneumatique avec ledit canal d'écoulement, chacune desdites première et seconde conduites de ramification ayant un volume de gaz prédéterminé et chacune desdites première et seconde conduites de ramification comprenant un canal de mesure (5) pour transmettre des signaux de pression gazeuse à partir dudit canal d'écoulement,
dans lequel ladite première conduite de ramification comprend au moins un capteur (9) en communication pneumatique avec ledit canal de mesure de la première conduite de ramification pour fournir un signal indicatif d'une pression dans ledit canal d'écoulement, dans lequel l'au moins un capteur (9) comprend un manomètre utilisable pour obtenir un signal indicatif d'une pression de voies respiratoires dans ledit canal d'écoulement (2), ledit agencement comprenant en outre un capteur de pression différentielle en communication pneumatique avec lesdits canaux de mesure desdites première et seconde conduites de ramification pour obtenir un signal indicatif d'un débit de gaz respiratoire dans ledit canal d'écoulement (2),
**caractérisé en ce que**
ladite seconde conduite de ramification comprend un volume d'équilibrage (11) en communication pneumatique avec la seconde conduite de ramification pour égaliser lesdits volumes de gaz prédéterminés desdites première et seconde conduites de ramification.

2. Agencement selon la revendication 1, dans lequel ledit volume d'équilibrage est configuré pour être intégré avec ou soit directement raccordable à soit indirectement raccordable à ladite seconde conduite de ramification, et ladite seconde conduite de ramification a un volume prédéterminé inférieur au volume prédéterminé de ladite première conduite de ramification.

3. Agencement selon la revendication 2, dans lequel ledit volume d'équilibrage égalisant lesdits volumes de gaz prédéterminés desdites première et seconde conduites de ramification est configuré pour ajouter le volume de gaz à ladite seconde conduite de ramification, ayant pour résultat le fait que le volume de gaz varie de moins de 10 %, plus spécifiquement de moins de 5 % ou encore plus spécifiquement de moins de 1 % par rapport à ladite première conduite de ramification.

4. Agencement selon la revendication 1, dans lequel ledit volume d'équilibrage est une chambre de gaz en communication pneumatique avec ladite seconde conduite de ramification.

5. Agencement selon la revendication 1, comprenant en outre le canal d'écoulement (2), dans lequel le canal d'écoulement (2) comprend un adaptateur d'écoulement (1) ayant un élément de restriction (4) pour restreindre l'écoulement à travers ledit adaptateur, lesdits canaux de mesure étant raccordés aux deux côtés dudit élément de restriction par l'intermédiaire d'entrées respectives de canal de mesure (7).

6. Agencement selon la revendication 1 ou 5, dans lequel le capteur de pression différentielle est destiné à obtenir un signal indicatif d'un écoulement de gaz respiratoire dans ledit canal d'écoulement (2) et le manomètre est destiné à obtenir un signal indicatif d'une pression de voies respiratoires dans ledit canal d'écoulement (2).

7. Agencement selon la revendication 6, dans lequel ledit volume d'équilibrage est destiné à compenser un volume interne (12) dudit manomètre.

8. Agencement selon la revendication 1, dans lequel lesdites première et seconde conduites de ramification (5) comprennent également au moins un composant pneumatique interne ayant une restriction principale (27), et dans lequel ladite restriction principale (27) est une valve de remise à zéro (10) destinée à remettre ledit capteur à la pression ambiante.

9. Agencement selon la revendication 8, dans lequel ladite seconde conduite de ramification a un volume plus petit dans ledit canal de mesure précédant ladite valve de remise à zéro que la première conduite de ramification.

10. Agencement selon la revendication 8, dans lequel ledit capteur est en communication pneumatique avec ledit canal d'écoulement (2) par le biais de ladite valve de remise à zéro.

11. Agencement selon la revendication 8, dans lequel chacune desdites au moins deux conduites de ramification (5) a une restriction principale identique (27).
